# EUROPEAN PATENT SPECIFICATION

(11) **EP 2 462 899 B1**
(45) Date of publication and mention of the grant of the patent: **12.11.2014**
(21) Application number: 10806658.0
(22) Date of filing: 05.08.2010
(51) Int. Cl.: A61L 27/56, A61F 2/28, A61C 8/00, A61L 27/36, A61F 2/46

(54) **ALVEOLAR BONE GRAFT PROCESSING METHOD AND ALVEOLAR BONE GRAFT THEREFROM**
VERFAHREN ZUR HERSTELLUNG EINES ALVEOLAREN KNOCHENIMPLANTATS, IN DIESEM VERFAHREN HERGESTELLTES ALVEOLARES KNOCHENIMPLANTAT
ROCÉDÉ DE RÉALISATION DE GREFFON OSSEUX ALVÉOLAIRE ET GREFFON OSSEUX ALVÉOLAIRE

(30) Priority: 06.08.2009 KR 20090072492
(43) Date of publication of application: 13.06.2012
(73) Proprietor: Um, In Woong, Seoul 136-044 (KR)
(72) Inventor: Um, In Woong, Seoul 136-044 (KR)
(74) Representative: ABG Patentes, S.L.
(86) International application number: PCT/KR2010/005142
(87) International publication number: WO 2011/016684

(56) References cited:
- WO-A1-2004/089432
- KR-A- 20020 011 164
- KR-A- 20040 065 203
- KR-A- 20040 087 438
- US-A- 5 112 354
- US-A- 5 112 354
- US-A- 6 089 867
- DATABASE WPI Week 200259 Thomson Scientific, London, GB; AN 2002-554970 XP002699579, & KR 2002 0011164 A (HUMAN TISSUE ENG CENT CO LTD) 8 February 2002 (2002-02-08)

## Description

### [Technical Field]

The present invention relates to an alveolar bone graft, and more particularly, to a method in which an extracted patient's own tooth or the same kind of tooth is physically and chemically processed into an alveolar bone graft having a block film form, a block plate form, and a tooth root form, the alveolar bone graft having the block film form, the block plate form, and the tooth root form, and a treatment method using the alveolar bone graft.

### [Background Art]

According to implant operations generalized already, now the trend is being changed to place an implant immediately after a tooth is extracted. In this case, it is necessary to preserve and reinforce the damaged alveolar bone.

Although various kinds of synthetic bones, xeno grafts, and allografts are used to preserve and reinforce an alveolar bone for implant operation, bone grafts superior to an autogenous bone do not exist. Most of grafts used at present is manufactured by utilizing an inorganic or organic matter of the autogenous bone or by synthesizing a portion of components of the autogenous bone to perform a portion of functions of the autogenous bone.

In a case of patients having congenital or acquired defects in alveolar bone, it may be difficult to solidly fix a fixture for implant operation. Thus, in this case, the implant operation may be performed after a bone graft made of others bone or artificial structure is implanted into the alveolar bone.

However, the bone graft made of others bone has limitations related to immunity. To solve the immunity-related limitations, complicated processing processes for the bone graft are required.

Also, since the conventional bone graft made of the artificial structure does not contain bone morphogenetic proteins, there is a limitation that a bone regeneration rate is low. Also, in case of the autogenous bone, there are limitations that an additional operation is needed, and an amount of raw material is limited. Document US 5,112,354 discloses an alveolar bone graft processing method starting from a bone from a patient. Document WO 2004/089432 discloses an alveolar bone graft processing method using teeth as starting material. Therefore, there is a great need for developing bone grafts which can solve the above-described limitations.

### [Disclosure]

### [Technical Problem]

To solve the above-described limitations, the present invention provides an alveolar bone graft in which an extracted patient's own tooth or the same kind of tooth is processed to be utilized for an implant operation and a processing method thereof.

The present invention also provides an alveolar bone in which patient's own tooth or the same kind of tooth is processed into a block film form, a block plate form, and a tooth root form and a processing method thereof.

The present invention also provides a treatment method which can treat defects of an alveolar bone using the above-described alveolar bone graft.

### [Technical Solution]

In accordance with an exemplary embodiment, an alveolar bone graft processing method includes: (a) separating a tooth gathered from a patient into a crown and a root; (b) trimming a soft tissue and enamel from the crown and the root; (c) cleaning the crown and root on which the step (b) is performed; (d) dewatering, degreasing and decalcifying the cleaned crown and root; and (e) freeze-drying the dewatered, degreased, and decalcified crown and root.

A plurality of through-holes may be formed in each surface of the crown and root cleaned through the step (c), and each of the through-holes may have a diameter of about 1 mm or less.

The freeze-dried crown and root may be sterilized.

The cleaning of the step (c) may include demineralized water-ultrasonic cleaning and hydrogen peroxide-ultrasonic cleaning.

The alveolar bone graft processing method may further include cleaning the crown and root dewatered, degreased, and decalcified in the step (d); and additionally dewatering and degreasing.

The dewatering of step (d) may be performed using ethyl alcohol, the degreasing of the step (d) may be performed using ethyl ether, and the decalcifying of the step (d) may be performed using hydrogen chloride.

The crown and root on which the stop (e) is performed may be processed into one of a block film, a block plate, and a tooth root.

The root having the root form which is not cut in the step (e) may be processed in the tooth root form.

The sterilizing may be performed using radiation or ethylene oxide gas.

The alveolar bone graft having the block film form may be manufactured by physically untwisting a fibroid material of the crown or root having the block film form; and compressing and processing the fibroid material to a predetermined thickness.

The alveolar bone graft may be processed through the above-described methods and applied to an implant operation.

In accordance with another exemplary embodiment, a treatment method using an alveolar bone graft, the treatment method includes: (a) separating a tooth gathered from a patient into a crown and a root; (b) processing the crown and the root so that only a dentin component remains by trimming a soft tissue and enamel; (c) cleaning the crown and the root on which the step (b) is performed; (d) dewatering, degreasing and decalcifying the cleaned crown and root; and (e) processing at least one of the dewatered, degreased, and decalcified crown and root into an alveolar bone graft having one of a block film, a block plate, and a tooth root; (f) freeze-drying and sterilizing the alveolar bone graft; and (g) implanting the alveolar bone graft into an alveolar bone of the patient to be implant-operated.

The block film may have a thickness of about 1 mm or less, and the block plate has a thickness of about 1 mm or more. The tooth root may have a thickness greater than that of the block plate.

The treatment method may further include physically untwisting fibroid materials of the crown and root having the block film form; and compressing the fibroid materials to a predetermined thickness to process the fibroid materials into the alveolar bone graft.

In accordance with further another exemplary embodiment, a treatment method using an alveolar bone graft, the treatment method include: (a) separating a tooth gathered from a patient into a crown and a root; (b) processing the root so that only a dentin component remains by trimming a soft tissue; (c) cleaning the root on which the step (b) is performed; (d) dewatering, degreasing and decalcifying the cleaned root; and (e) processing the dewatered, degreased, and decalcified root into an alveolar bone graft; (f) freeze-drying and sterilizing the alveolar bone graft; and (g) implanting the alveolar bone graft into an alveolar bone of the patient to be implant-operated.

### [Advantageous Effects]

According to the present invention, the alveolar bone graft may be processed into the block film form, the block plate form, or the tooth root form using the extracted own tooth and the treatment method using the alveolar bone graft may be provided. Thus, an amount of bone transplantation material may be increased, and the implant operation may be stably performed. In addition, since the own tooth is used, the metastasis of infectious diseases of the xeno (animal) graft may be prevented and the autogenous bone graft may be possible without additional operations, thereby obtaining the same effects.

Also, since the own tooth is used, the alveolar bone graft according to the present invention may be stably and economically operated without immune rejection response.

### [Description of Drawings]

FIG. 1 is a view of a tooth divided into a crow and a root.
FIG. 2 is a view of a crown in which only dentin remains by completely trimming enamels and soft tissues within neurons.
FIG. 3 is a view of a crown having a plurality of through-holes.
FIG. 4 is a view of a root in which only a dentin remains by completely trimming enamels and soft tissues within neurons.
FIG. 5 is a view of a crown having a plurality of through-holes.
FIG. 6 is a view of an alveolar bone graft in which a crown is cut into block films using a microtome knife.
FIG. 7 is a view of an alveolar bone graft in which a crown is cut into block plates using a microtome knife.
FIG. 8 is a view of an alveolar bone graft in which a root is cut into a block film using a microtome knife.
FIG. 9 is a view of an alveolar bone graft in which a root is cut into a block plate using a microtome knife.
FIG. 10 is a view of an alveolar bone graft having a tooth root form.
FIG. 11 is a flowchart for explaining an alveolar bone graft processing method and an implant operation applying process.
FIG. 12 is a flowchart for explaining an operation applying process to which cleaning, dewatering, degreasing, and decalcifying processes are added in the alveolar bone graft processing method and the implant operation applying process.
FIG. 13 is a flowchart for explaining a processing method of an alveolar bone graft having a tooth root form among alveolar bone grafts and an implant operation applying process.

### [Mode for Invention]

The present invention relates to an alveolar bone graft which may be adequately used for each place and use using own teeth or the same kind of tooth, a processing method thereof, and a treatment method using the same. The alveolar bone graft according to the present invention may be processed into a block film form, a block plate form, or a tooth root form. That is, the alveolar bone graft may be processed into a block film form, a block plate form, and a tooth root form using a microtome knife according to a shape and form of a defect portion.

Components of a tooth may be nearly similar to that of an autogenous bone. However, the tooth may be slightly different for each place in component and physical property. Thus, the tooth may be differently processed for each place and adequately used for a use of autogenous bone graft. An enamel and dentin may be available in the tooth. In the current embodiment, an alveolar bone graft, in which the enamel is trimmed, using the dentin will be described.

Hereinafter, a method for the alveolar bone graft into a block film form, a block plate form, and a tooth root form will be described in detail.

First, in operation S100, a tooth that is a basic material is extracted from a patient. The extracted tooth is kept in a cold place in a state where the tooth is immersed into alcohol, saline solution, or distilled water, etc.

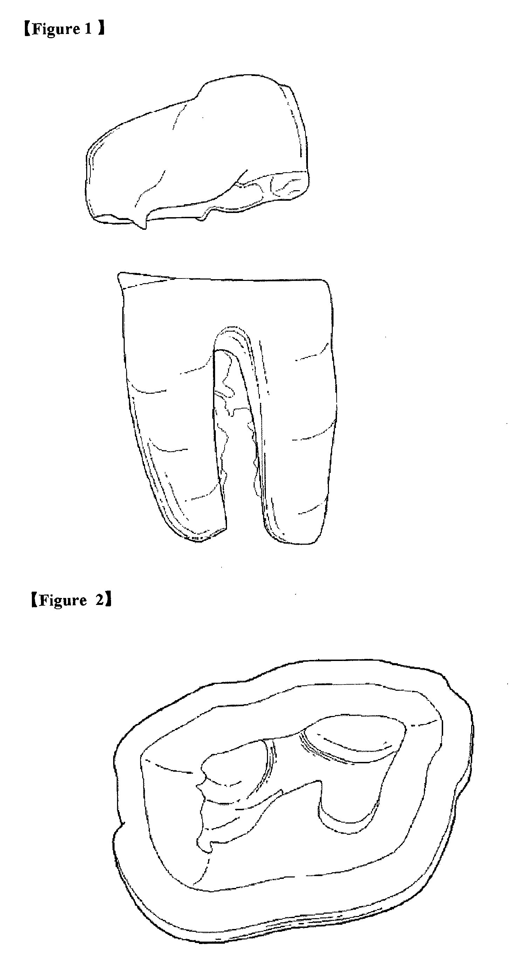

In operation S200, the tooth kept in the cold place is cut at a cemento enamel junction (CEJ) between a crown (a head portion) and a root (a root portion) and separated from each other. Referring to FIG. 1, a state in which the tooth is divided into the crown and the root may be observed.

In operation S300, the enamel and soft tissues within neurons may be completely trimmed from the crown to allow only the dentin to remain. Referring to FIG. 2, the crown on which only the dentin remains may be observed.

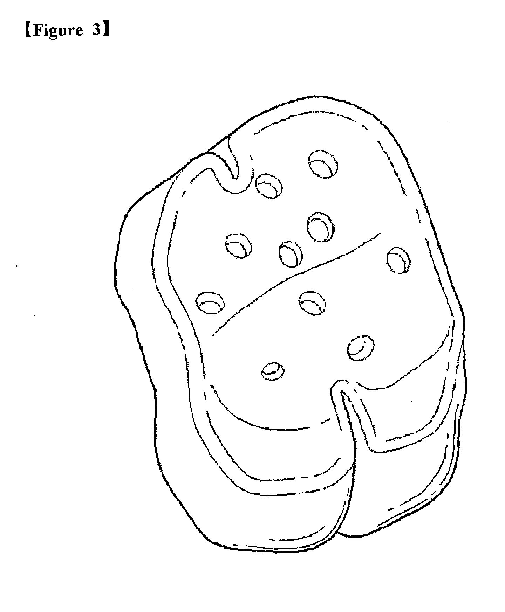

After the crown including only the dentin is cleaned in operation S400, a plurality of through holes having a diameter of about 1 mm or less are punched in each surface of the crown in operation S500. Referring to FIG. 3, the crown having the plurality of through-holes may be observed.

The same process as that of the crown may be performed on the root.

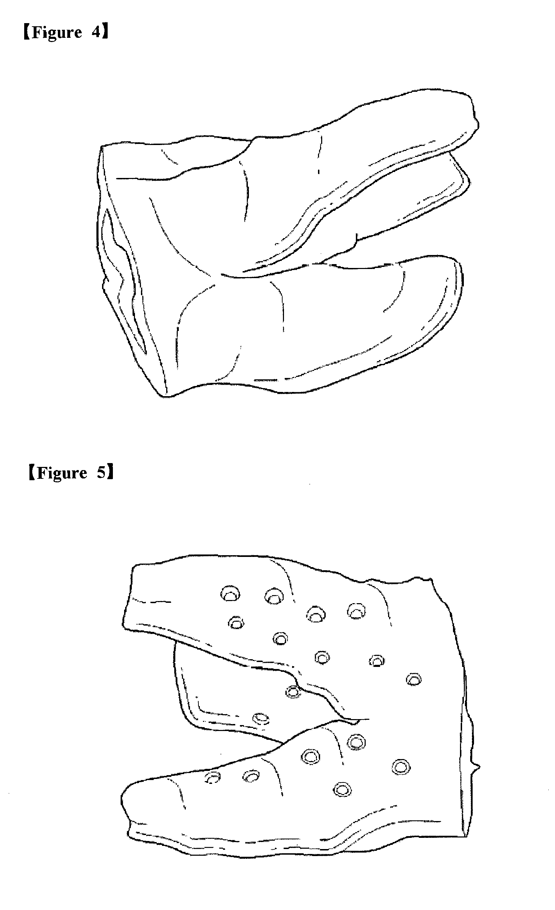

In operation S300, soft tissues and nervous tissues are trimmed from the root. Referring to FIG. 4, the root in which the soft tissues and nervous tissues are trimmed to remain dentin may be observed.

An end of the root trimmed through the above-described methods is horizontally cut by about 1 mm to about 2 mm and removed so that the end of the root is flat. Then, in operation S500, the root is punched with a certain interval to form holes having a small diameter of about 1 mm. Referring to FIG. 5, the root having a plurality of through-holes may be observed.

The removing of the end of the root is for the reason in which the alveolar bone graft is mounted at a correct position when the alveolar bone graft is applied to an implant operation to prevent the alveolar bone graft from having an influence on the outside (surrounding bones).

The plurality of through-holes is for securing penetration spaces of blood vessels. That is, effector mechanisms of the graft contain inorganic and organic matters (e.g., collagen fibers and proteins) of the autogenous bone. Thus, when it is intended to allow the effector mechanisms to act, the adequate supply of blood is of the highest priority. Thus, it is necessary to secure a fine space for supplying the blood into the graft having the tooth root form (or the block film form or the block plate form) maintained with a predetermined volume. For this, the plurality of through-holes are formed. A plurality of holes having a diameter of about 0.25 mm to about 1.00 mm is formed inward from the outside of the tooth root using a specific mechanism (e.g., physical mechanism) to form the through-holes. A distance between the holes may range from about 1 mm to about 2 mm. The number of holes may be varied according to a surface area of the tooth root. Generally, in case of a bicuspid tooth of an adult, about 40 to 50 holes may be formed. In case of a molar tooth, about 50 to 70 holes may be formed.

The crown and root may be cleaned through the above-described processes to trim contaminants and remaining soft tissues.

In operation S400, the crown and root may be immersed into demineralized water or hydrogen peroxide solution to clean the crown and root using an ultrasonic cleaner. First, the crown and root are cleaned three times for about 5 minutes to about 10 minutes each time using the demineralized water-ultrasonic cleaner, and then are cleaned three times for about 10 minutes to about 30 minutes each time using the hydrogen peroxide-ultrasonic cleaner. Thereafter, the crown and root are cleaned again three times for about 5 minutes to about 10 minutes each time using the demineralized water-ultrasonic cleaner. Here, the hydrogen peroxide solution may have a concentration of about 5% to about 7%. Also, sterile distilled water may be replaced with the demineralized water. Fat floating upward during the cleaning process may be cleaned using ether. The cleaning solution may have a volume greater than about 5 times to 10 times of that of the dentin.

When the cleaned crown and root are dewatered and degreased in operation S600, the crown and root may be easily disinfected, and the possibility of contamination may be reduced. When the dewatering and degreasing processes are performed, the fat of the crown and root may be removed. Thus, only the inorganic matter, the organic matter, and the collagen that are advantage to form the bone may remain to serve as the graft which does not have immune rejection response at all. The dewatering process may be performed for about 10 minutes to about 1 hour with ethanol (ethyl alcohol) having a volume of about 5 times to 10 times of those of the root and dentin of the cleaned crown. The degreasing process may be performed for about 10 minutes to about 1 hour with ethyl ether having a volume of about 5 times to about 10 times of those of the dewatered crown and root.

The crown and root may be decalcified within about 0.2N to about 0.7N HCl (a volume of about 20 times) solution for about 12 hours to about 72 hours. An amount of calcium remaining after the decalcifying may be less than about 8%. Here, about 5 ml of HCl solution per about 1 g of dentin may be used.

In operation S610, the decalcified crown and root may be additionally cleaned three times for about 5 minutes to about 10 minutes each time using demineralized water-ultrasonic cleaner. In operation S620, after the additional cleaning process is performed, the crown and root may be additionally dewatered and decalcified. The additional dewatering process may be performed using ethanol for about 10 minutes to about 1 hour, and the additional decalcifying process may be performed using ethyl ether for about 10 minutes to about 1 hour.

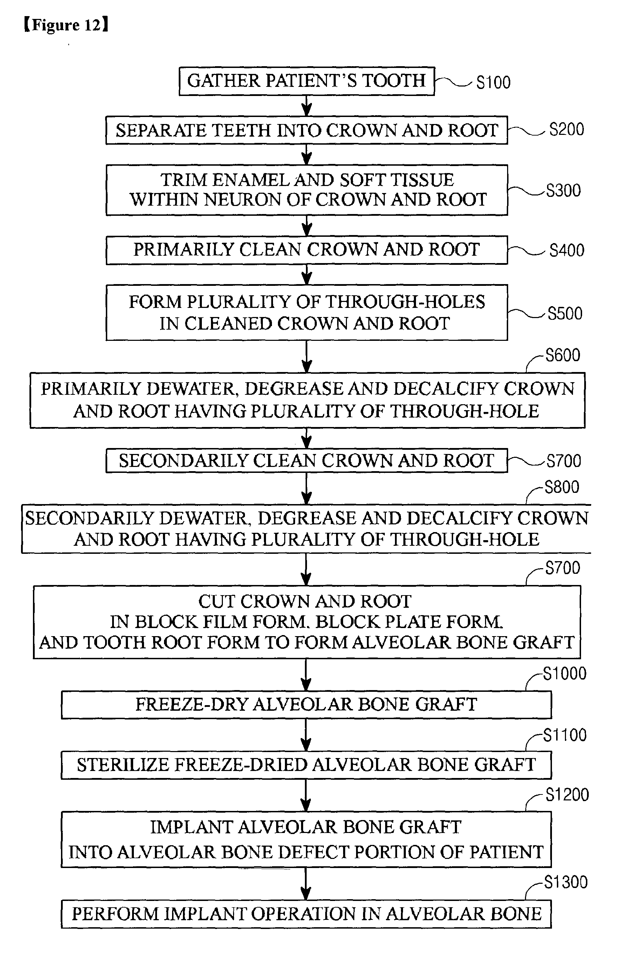

Referring to FIG. 12, a process for applying the alveolar bone graft, in which the additional cleaning, dewatering, and degreasing processes are performed, to the implant operation will be described.

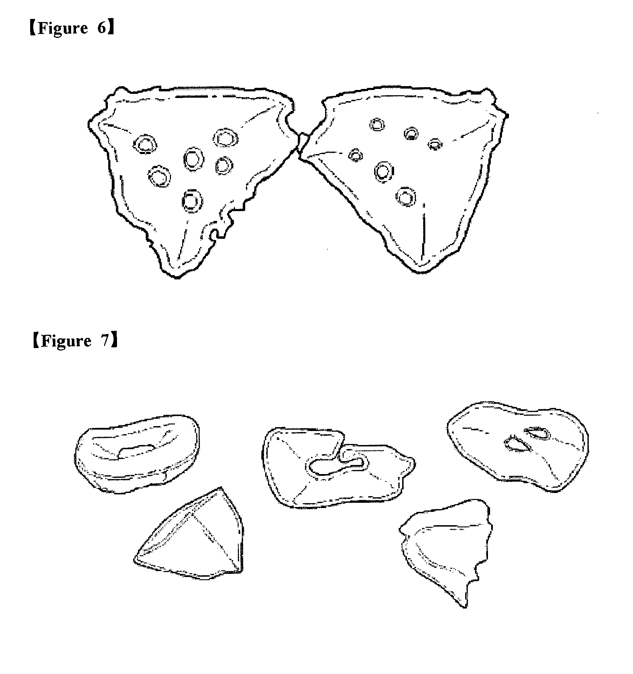

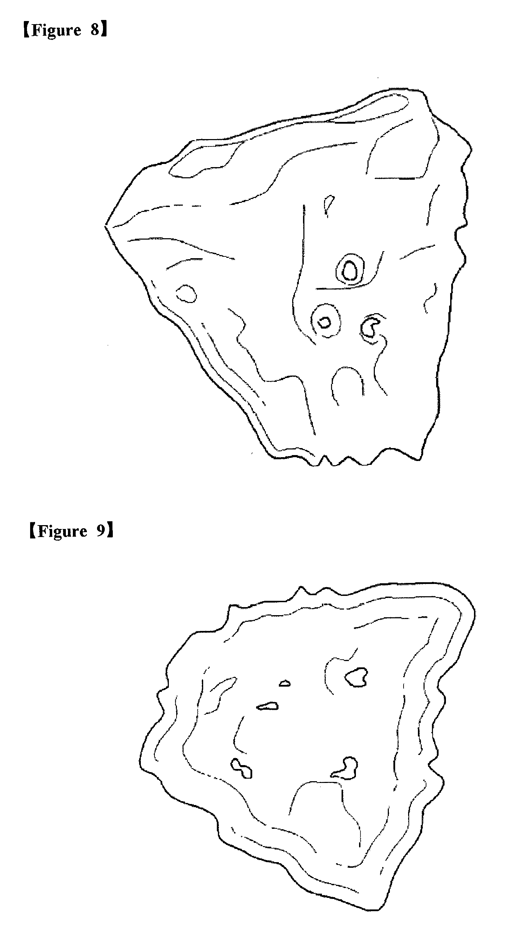

In operation S700, the crown and root are cut at thicknesses of about 0.2 mm, 0.4 mm, 0.6 mm, and 0.8 mm (various thicknesses of about 1 mm or less) using the microtome knife to process the crown and root into the block films. Referring to FIG. 6, the block film formed by cutting the crown using the microtome knife may be observed. Referring to FIG. 8, the block film formed by cutting the root using the microtome knife may be observed. The plurality of holes may be observed in the block film. The present invention is not limited to a shape of the block film. For example, the block film may be changed in shape according to the shape and form of the defect portion.

When the graft is formed using the block film, a fibroid material may be untwisted through a physical method. This is possible because the remaining amount of calcium is reduced by the decalcifying process. The fibroid material may be processed at a certain thickness using a physical machine. The processed block film may be cut adequate for a standard to manufacture and process the block film adequate for the defect portion.

The physical machine may be a machine using compression and pressure and thus include a roller or press.

In operation S700, the crown and root may be cut at thicknesses of about 1.0 mm, 1.5 mm, 2.0 mm, 3.0 mm, 4.0 mm, and 5.0 mm to process the crown into the block plate. Referring to FIG. 7, the crown cut in the block plate form using the microtome knife may be observed. Also, referring to FIG. 9, the root cut in the block plate form may be observed. Also, the porous block plate may be observed. The present invention is not limited to a shape of the block plate. For example, the block plate may be changed in shape according to the shape and form of the defect portion. The alveolar bone graft may be manufactured in the tooth root form having a thickness greater than those of the block film form and the block plate form. The tooth root may be changed in shape and form according to a shape and form of the defect portion.

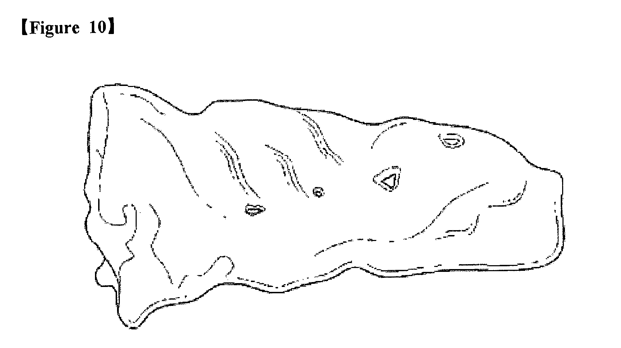

When the alveolar bone graft is manufactured in the tooth root form, the root may be used as it is. That is, in operation S700, the root may be implanted in its original position to restore the damaged alveolar bone in its original shape. In this manufacturing method, the nervous and soft tissues within the root separated from the tooth may be trimmed in operation S300. Then, the plurality of through-holes having a diameter of about 1 mm or less are formed in operation S500. In operation S600, the dewatering, degreasing, and decalcifying processes may be performed. Thereafter, the root may be decalcified again within a hydrochloric acid solution, in which about 0.2N to about 0.7N HCl is dissolved, for about 12 hours to about 72 hours. Then, the cleaning process may be performed again to complete the alveolar bone graft. Unlike the block film and the block plate, the root form which is not cut may be implanted in an original position at which the root is extracted to restore the alveolar bone or may be mounted on the implant. Referring to FIG. 10, the alveolar bone graft manufactured in the tooth root form may be observed.

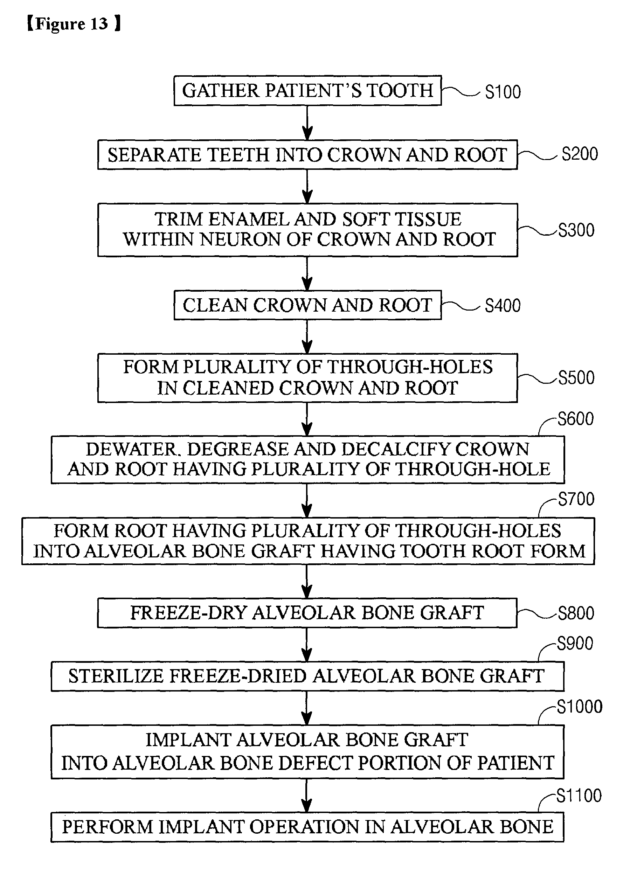

Referring to FIG. 13, a method for processing the alveolar bone graft having the tooth root form and an implant operation process using the same will be described.

As necessary, the block film, the block plate, and the tooth root may be cut in various directions to manufacture the alveolar bone graft. The cutting direction may be a horizontal or vertical direction according to a use of the alveolar bone graft. In operation S800, the block film graft having a predetermined shape is freeze-dried. Thus, physical properties of the tissues may be maintained, and also a content of moisture may be reduced through the freeze-drying.

In operation S900, after the freeze-drying, a sterilization process may be further performed before the implant operation. In case of the freeze-dried tooth, the tooth may be applied to a patient's alveolar bone to be implanted in operation S1000. Also, in operation S1100, the tooth may be utilized in the implant operation process.

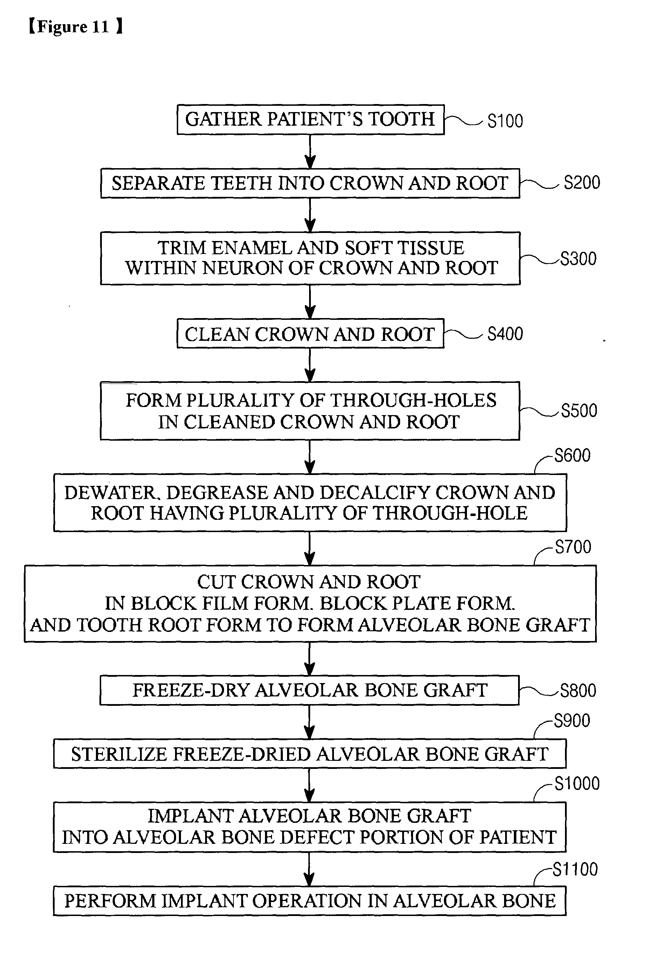

Referring to FIG. 11, a method for applying the alveolar bone graft according to the present invention to the implant operation will be described.

The present invention relates to a technique in which a tooth discarded after extraction is converted into the alveolar bone graft having the same function and effect as those of the autogenous bone graft. In case of the bone film according to the present invention, since the bone film may contain the same collagen and bone formation induction protein as that of the natural autogenous bone and also has absorption ability for bone remolding, the bone film is the best suitable for existing guided bone regeneration. Also, in case of the bone piece (bone block), the bone block may have ability of guided bone regeneration and vertical alveolar bone augmentation at the same time. Also, in case of the root, the root may have ability in which the damaged toot is regenerated in its original shape.

Also, the teeth may have the nearly same component as that of bone. Specifically, the tooth may have the nearly same component as those of the alveolar bone, the soft tissue, and hard tissue. Thus, the present invention may perform the same function as that of the autogenous bone graft. Also, since the hard tissue and soft tissue of the tooth may be preserved and bone formation factors of the teeth have the same component and effect, if they preserve their functions, the alveolar bone graft may perform the same function as that of the autogenous bone graft. Thus, if the alveolar bone graft is adequately sterilized, the immune rejection response may be prevented (because of autogenous tooth tissues).

Thus, according to the present invention, limitations occurring in general grafts, i.e., a metastasis of infectious diseases of xeno (animal) graft, an inflammation, etc, may be prevented and also partially localized functions may be complemented to obtain the stable alveolar bone graft having the same function as that of the autogenous bone graft.

Also, according to the present invention, the discarded tooth may be used as the alveolar bone graft having the same function as that of the autogenous bone graft without additional operations to solve limitations in quantity and quality at the same time.

Also, according to the present invention, the freeze-dried alveolar bone graft may be sterilized using radiation or ethylene oxide gas. Thus, since remaining hydroxyapatite, organic collagen, and bone formation factors may be used, the alveolar bone graft may be functions more similar to that of an autogenous bone.

According to the present invention including the above-described processes, an amount of bone transplantation material for implanting own tooth or the same kind of tooth may be increased, and the implant operation may be stably performed. Also, since the own tooth or the same kind of tooth is used, the metastasis of infectious diseases of the xeno (animal) graft may be prevented and the autogenous bone graft may be possible without additional operations, the alveolar bone graft may be stably and economically operated without immune rejection response.

## Claims

1. An alveolar bone graft processing method comprising:
(a) separating a tooth gathered from a patient into a crown and a root;
(b) processing the crown, root, or crown and root so that only a dentin component remains by trimming a soft tissue and enamel;
(c) cleaning the crown, root, or crown and root on which the step (b) is performed;
(d) forming a plurality of through-holes in each surface of the crown, root, or crown and root cleaned through the step (c);
(e) dewatering, degreasing and decalcifying the cleaned crown, root, or crown and root;
(f) processing the dewatered, degreased, and decalcified crown, root, or crown and root into one of a block film, a block plate, and a tooth root; and
(g) freeze-drying the dewatered, degreased, and decalcified crown, root, or crown and root.

2. The alveolar bone graft processing method according to Claim 1, wherein each of the through-holes has a diameter of about 1 mm or less.

3. The alveolar bone graft processing method according to Claim 1, further comprising horizontally removing an end of the root by 1 mm to 2 mm.

4. The alveolar bone graft processing method according to Claim 1, further comprising sterilizing the crown, root, or crown and root on which the step (g) is performed.

5. The alveolar bone graft processing method according to Claim 4, wherein the sterilizing of the crown, root, or crown and root is performed using radiation or ethylene oxide gas.

6. The alveolar bone graft processing method according to Claim 1, further comprising cleaning the crown, root, or crown and root dewatered, degreased, and decalcified in the step (e); and
additionally dewatering and degreasing the crown, root, or crown and root.

7. The alveolar bone graft processing method according to Claim 1, wherein the dewatering of the crown, root, or crown and root in step (e) is performed using ethyl alcohol.

8. The alveolar bone graft processing method according to Claim 1, wherein the degreasing of the crown, root, or crown and root in the step (e) is performed using ethyl ether.

9. The alveolar bone graft processing method according to Claim 1, wherein the decalcifying of the crown, root, or crown and root in step (e) is performed using hydrochloric acid.

10. The alveolar bone graft processing method according to Claim 1, wherein the block film has a thickness of about 1 mm or less, the block plate has a thickness of about 1 mm or more, and the tooth root has a thickness greater than that of the block plate.

11. The alveolar bone graft processing method according to Claim 1, further comprising physically untwisting a fibroid material of the crown, root, or crown and root having the block film form; and
compressing and processing the fibroid material to a predetermined thickness.

12. An alveolar bone graft processed using the methods of Claim 1, 6 or 11.

## Patentansprüche

1. Verfahren zur Verarbeitung eines Alveolarknochentransplantats umfassend:
(a) Trennen eines vom Patienten gewonnenen Zahns in Krone und Wurzel;
(b) Verarbeiten der Krone, Wurzel oder Krone und Wurzel, so dass nur die Zahnbeinbestandteile durch Entfernung des Weichteilgewebes und des Schmelzes übrig bleiben;
(c) Säubern der Krone, der Wurzel oder der Krone und Wurzel bei welchen Schritt (b) durchgeführt wurde;
(d) Ausgestalten einer Vielzahl von durchgängigen Löchern in jeder Oberfläche der durch Schritt (c) gesäuberten Krone, Wurzel oder Krone und Wurzel;
(e) Entwässerung, Entfettung und Dekalzifizierung der gereinigten Krone, Wurzel oder Krone und Wurzel;
(f) Verarbeiten der entwässerten, entfetteten und dekalzifizierten Krone, Wurzel oder Krone und Wurzel zu einem Blockfilm, einer Blockplatte oder einer Zahnwurzel; und
(g) Gefriertrocknen der entwässerten, entfetteten und dekalzifizierten Krone, Wurzel oder Krone und Wurzel.

2. Verfahren zur Verarbeitung eines Alveolarknochentransplantats nach Anspruch 1, wobei jedes der durchgängigen Löcher einen Durchmesser von etwa 1 mm oder weniger aufweist.

3. Verfahren zur Verarbeitung eines Alveolarknochentransplantats nach Anspruch 1, weiterhin umfassend das horizontale Entfernen eines Endes der Wurzel um 1 bis 2 mm.

4. Verfahren zur Verarbeitung eines Alveolarknochentransplantats nach Anspruch 1, weiterhin umfassend das Sterilisieren der Krone, Wurzel oder Krone und Wurzel bei der Schritt (g) ausgeführt wurde.

5. Verfahren zur Verarbeitung eines Alveolarknochentransplantats nach Anspruch 4, wobei die Sterilisierung der Krone, Wurzel oder Krone und Wurzel mittels Strahlung oder Ethylenoxidgas durchgeführt wird.

6. Verfahren zur Verarbeitung eines Alveolarknochentransplantats nach Anspruch 1, weiterhin umfassend die Säuberung der in Schritt (e) entwässerten, entfetteten und dekalzifizierten Krone, Wurzel oder Krone und Wurzel; und
zusätzlichem Entwässern und Entfetten der Krone, Wurzel oder Krone und Wurzel.

7. Verfahren zur Verarbeitung eines Alveolarknochentransplantats nach Anspruch 1, wobei das Entwässern der Krone, Wurzel oder Krone und Wurzel in Schritt (e) mittels Ethylalkohol durchgeführt wird.

8. Verfahren zur Verarbeitung eines Alveolarknochentransplantats nach Anspruch 1, wobei das Entfetten der Krone, Wurzel oder Krone und Wurzel in Schritt (e) mittels Ethylether durchgeführt wird.

9. Verfahren zur Verarbeitung eines Alveolarknochentransplantats nach Anspruch 1, wobei das Dekalzifieren der Krone, Wurzel oder Krone und Wurzel in Schritt (e) mittels Salzsäure durchgeführt wird.

10. Verfahren zur Verarbeitung eines Alveolarknochentransplantats nach Anspruch 1, wobei der Blockfilm eine Dicke von etwa 1 mm oder weniger, die Blockplatte eine Dicke von etwa 1 mm oder mehr und die Zahnwurzel eine Dicke größer als die der Blockplatte aufweist.

11. Verfahren zur Verarbeitung eines Alveolarknochentransplantats nach Anspruch 1, weiterhin umfassend das physikalische Aufdrehen des faserartigen Materials der Krone, Wurzel oder Krone und Wurzel in Form des Blockfilms; und
Komprimieren und Verarbeiten des faserartigen Materials auf eine vorbestimmte Dicke.

12. Alveolarknochentransplantat verarbeitet mittels der Verfahren nach Ansprüchen 1, 6 oder 11.

## Revendications

1. Procédé de traitement de greffe osseuse alvéolaire comprenant :
(a) de séparer une dent prélevée d'un patient en une couronne et une racine ;
(b) de traiter la couronne, la racine ou la couronne et la racine de sorte que seule reste une composante de la dentine en dégageant le tissu mou et l'émail ;
(c) de nettoyer la couronne, la racine ou la couronne et la racine sur laquelle l'étape (b) est réalisée ;
(d) de former une pluralité de trous traversants dans chaque surface de la couronne, de la racine ou de la couronne et de la racine nettoyées à l'étape (c) ;
(e) de déshydrater, dégraisser et décalcifier la couronne, la racine ou la couronne et la racine nettoyées ;
(f) de traiter la couronne, la racine ou la couronne et la racine déshydratées, dégraissées et décalcifiées dans un élément choisi parmi un film de bloc, une plaque de bloc et une racine d'une dent ; et
(g) de lyophiliser la couronne, la racine ou la couronne et la racine déshydratées, dégraissées et décalcifiées.

2. Procédé de traitement de greffe osseuse alvéolaire selon la revendication 1, dans lequel chacun des trous traversants a un diamètre d'environ 1 mm ou moins.

3. Procédé de traitement de greffe osseuse alvéolaire selon la revendication 1, comportant en outre d'éliminer horizontalement une extrémité de la racine sur 1 mm à 2 mm.

4. Procédé de traitement de greffe osseuse alvéolaire selon la revendication 1, comportant en outre de stériliser la couronne, la racine ou la couronne et la racine sur lesquelles l'étape (g) est réalisée.

5. Procédé de traitement de greffe osseuse alvéolaire selon la revendication 4, dans lequel la stérilisation de la couronne, de la racine, ou de la couronne et de la racine est réalisée en utilisant un rayonnement ou un gaz d'oxyde d'éthylène.

6. Procédé de traitement de greffe osseuse alvéolaire selon la revendication 1, comportant en outre de nettoyer la couronne, la racine, ou la couronne et la racine déshydratées, dégraissées et décalcifiées à l'étape (e) ; et
de déshydrater et de dégraisser en outre la couronne, la racine, ou la couronne et la racine.

7. Procédé de traitement de greffe osseuse alvéolaire selon la revendication 1, dans lequel la déshydratation de la couronne, de la racine, ou de la couronne et de la racine à l'étape (e) est réalisée en utilisant de l'alcool éthylique.

8. Procédé de traitement de greffe osseuse alvéolaire selon la revendication 1, dans lequel le dégraissage de la couronne, de la racine, ou de la couronne et de la racine à l'étape (e) est réalisée en utilisant de l'éther éthylique.

9. Procédé de traitement de greffe osseuse alvéolaire selon la revendication 1, dans lequel la décalcification de la couronne, de la racine, ou de la couronne et de la racine à l'étape (e) est réalisée en utilisant de l'oxyde hydrochlorhydrique.

10. Procédé de traitement de greffe osseuse alvéolaire selon la revendication 1, dans lequel le film de bloc a une épaisseur inférieure ou égale à environ 1 mm, la plaque de bloc a une épaisseur supérieure ou égale à environ 1 mm, et la racine de la dent a une épaisseur supérieure à celle de la plaque de bloc.

11. Procédé de traitement de greffe osseuse alvéolaire selon la revendication 1, comportant en outre de dérouler physiquement un matériau fibrome de la couronne, de la racine, ou de la couronne et de la racine ayant la forme du film de bloc ; et
de comprimer et de traiter le matériau fibrome selon une épaisseur prédéterminée.

12. Greffe osseuse alvéolaire qui a été traitée en utilisant les procédés de la revendication 1, 6 ou 11.
